Europäisches Patentamt

European Patent Office

Office européen des brevets

(11) Publication number: **0 079 210**
**B1**

(12) **EUROPEAN PATENT SPECIFICATION**

(45) Date of publication of patent specification: **13.08.86**

(51) Int. Cl.⁴: **G 01 N 21/35, G 01 N 33/30**

(21) Application number: **82305889.6**

(22) Date of filing: **05.11.82**

(54) **Device for determining the soot content of an oil sample.**

(30) Priority: **07.11.81 DE 8132595 u**

(43) Date of publication of application:
**18.05.83 Bulletin 83/20**

(45) Publication of the grant of the patent:
**13.08.86 Bulletin 86/33**

(84) Designated Contracting States:
**BE DE FR GB IT NL**

(56) References cited:
**DE-A-2 158 007**
**DE-A-2 745 483**

**HOUBEN-WEYL "Methoden der organischen
Chemie", 4th edition, vol. III, part 2, 1955,
GEORG THIEME VERLAG, Stuttgart**

(73) Proprietor: **MOBIL OIL CORPORATION**
**150 East 42nd Street**
**New York New York 10017 (US)**

(72) Inventor: **Hohensang, Lutz**
**Wolliner Strasse 56**
**D-2000 Hamburg 73 (DE)**

(74) Representative: **West, Alan Harry et al**
**Mobil Court 3 Clements Inn**
**London WC2A 2EB (GB)**

Courier Press, Leamington Spa, England.

EP 0 079 210 B1

## Description

This invention relates to a device for determining the soot content of an oil sample.

Soot plays a decisive role in the service life of engine oil. An engine oil can only hold a certain amount of soot and if the oil change is not timely, the oil detergent additive overloaded with soot is deposited in the engine while it is not operating, and forms a gummy substance with the soot which no longer dissolves when the engine is reactivated. Under these conditions, damage to the engine appears within a relatively short time. The amount of soot produced depends on many factors, for example, engine type, manner of operation and engine setting.

Thus an effective oil analysis technique to determine soot content in engine oil and, ultimately, engine condition, is desirable in order to optimize oil, engine and filter life and to minimize maintenance costs. An effective oil analysis technique can also be used to determine the causes of various engine problems which may be encountered and is therefore useful in diagnosing engine problems and trends. Engine malfunctions which are determined in the analysis can be corrected early through minor maintenance and with minimum cost before the malfunction worsens.

For many years, attempts have been made to make available to engine operators a measuring device which could provide an easy yet reliable determination of the soot content of engine oil and also permit an estimation of the length of the remaining service life of the oil. Such a device must be easy to handle by automotive repair shops and construction site personnel and have independent power means. To date, however, available devices have not fulfilled these objectives.

Sophisticated and versatile monitoring devices have been devised, such as the device shown in DE—A1—2 158 007. The device is capable of analyzing samples which respond to either ultraviolet or infra-red radiation, and performing quite sophisticated measurements upon fluids flowing through the analyzer. An elaborate optical system comprising a number of lenses, and continuous monitoring devices are specified.

In Houben-Weyl: "Methoden der organischen Chemie" 4th Edition, Volume III, Part 2, 1955 at page 830 are disclosed infra-red — spectroscopic analytical techniques suitable for use in a laboratory.

In accordance with the invention there is now provided a device for determining the soot content of an oil sample comprising:

a receptacle (6) comprising two opposed, parallel wall members (4, 5) made of infra-red radiation transparent material defining a passage for receiving an oil sample;

a source (2, 3) of infra-red radiation disposed on one side of the receptacle adjacent one wall member (4); and

an infra-red radiation detector (10) disposed on the other side of the receptacle adjacent the other wall member (5) characterised in that:

the source (2, 3) comprises a filament bulb, one end of whose glass envelope is open, the open end being secured to the one wall member (4) such that the latter closes the glass envelope.

In the device of the invention, the bulb of an incandescent lamp defining the infra-red source is combined with the oil-sample receptacle so that part of the bulb also defines a side of the sample receptacle. In this way, short optical paths are ensured between the radiation source and the detector so as to enable a low power source, such as a flashlight battery, to be used to energise the infra-red source.

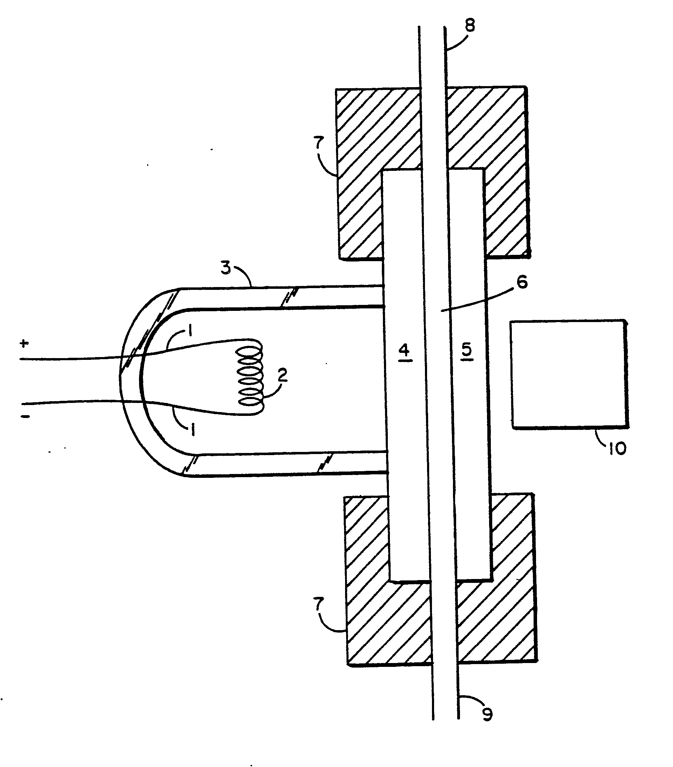

The accompanying drawing is a schematic representation of a device according to one example of the invention.

Referring to the drawing, the device includes a filament 2 which is supplied with current through wires 1 so that it emits infra-red radiation of a specific frequency. The filament 2 is surrounded by a bulb body 3 through one end of which pass the wires 1 and which at its other end includes an infra-red permeable window defined by one side wall 4 of a rest receptacle 6. The opposite side wall 5 of the receptacle 6 is also formed of an infra-red permeable material so that infra-red radiation emanating from the filament 2 can reach an infra-red detector 10 disposed adjacent the side wall 5. The distance between the side walls 4, 5 is kept at a small fixed value by spacers (not shown).

Inlet and outlet conduits 8, 9 respectively carried by identical mounting sections 7 communicate with the receptacle 6 to allow an oil sample to be introduced into the receptacle.

Any convenient measuring unit (not shown) is connected to, and receives, electrical signals from, the detector 10 to generate an output according to the magnitude of the signal from the detector. The output from the measuring unit then serves to inform a service attendant whether an oil sample being tested is capable of further service. A reading from the measuring unit can also be used to make an estimate of the remaining oil service life.

In use, a detergent is introduced into the receptacle 6 through the oil inlet 8 in order to remove any oil residues from the receptacle. The oil to be tested is then introduced through the inlet 8, the test quantity of oil typically being a few milliliters. The filament 2 is then connected to a power source which preferably is a low voltage battery (typically 6 or 8 volts), which may be built into the device. Infra-red radiation emitted by the filament 2 then passes through the side wall 4, the oil sample and the side wall 5 to the detector 10, which gives a reading corresponding to the soot content of the oil sample in the test. In this respect, it is to be appreciated that, since soot does not have well-defined infra-red absorption bands, but rather absorbs over a range of frequencies, gratings or prisms are not necessary when testing an oil sample.

The device may be constructed as a fully encapsulated unit in which only the oil inlet 8 and oil outlet 9 are open to the outside. An on-off switch may be connected in the circuit to the filament 2 and the reading from the measuring unit may be provided either in digital or analog form.

Any infra-red permeable material may be used for the side walls 4, 5, although calcium fluoride is preferred. The side wall 4 may be attached to the remainder of the bulb body, which is conveniently the glass bulb of a conventional incandescent lamp, in any convenient manner, for example by glueing or cementing.

The mounting sections 7 can be made of any suitable metal and are so constructed that changes in the distance between the side walls 4, 5 are precluded.

**Claims**

1. A device for determining the soot content of an oil sample comprising:
a receptacle (6) comprising two opposed, parallel wall members (4, 5) made of infra-red radiation transparent material defining a pasage for receiving an oil sample;
a source (2, 3) of infra-red radiation disposed on one side of the receptacle adjacent one wall member (4); and
an infra-red radiation detector (10) disposed on the other side of the receptacle adjacent the other wall member (5) characterised in that:
the source (2, 3) comprises a filament bulb, one end of whose glass envelope is open, the open end being secured to the one end of wall member (4) such that the latter closes the glass envelope.

2. A device according to claim 1, wherein the wall members (4, 5) of the receptacle (6) are formed of calcium fluoride.

3. A device according to claim 1 or claim 2, including a housing for a low voltage supply for powering the source (2, 3) of infra-red radiation.

**Patentansprüche**

1. Vorrichtung zum Bestimmen des Rußgehalts einer Ölprobe, die aufweist,
einen Behälter (6) mit zwei einander gegenüberliegenden parallelen Wandteilen (4, 5) aus einem für Infrarotstrahlung durchlässigen Material, die einen Durchgang für die Aufnahme einer Ölprobe festlegen;
eine Quelle (2, 3) für Infrarotstrahlung, die an einer Seite des Behälters neben einem Wandteil (4) angeordnet ist; und
einem Detektor (10) für Infrarotstrahlung, der an der anderen Seite des Behälters neben dem anderen Wandteil (5) angeordnet ist, dadurch gekennzeichnet, daß die Quelle (2, 3) einen Glühdrahtkolben aufweist, bei dem das eine Ende der Glashülle offen und an dem einen Wandteil (4) derart befestigt ist, daß das letztere die Glashülle verschließt.

2. Vorrichtung nach Anspruch 1, worin die Wandteile (4, 5) des Behälters (6) aus Calciumfluorid gebildet sind.

3. Vorrichtung nach Anspruch 1 oder 2, die ein Gehäuse für eine Niederspannungsquelle für die Energieversorgung der Quelle (2, 3) für die Infrarotstrahlung aufweist.

**Revendications**

1. Appareil pour déterminer la teneur en noir de fumée d'un échantillon d'huile, comprenant:
un récipient (6) possédant deux éléments de paroi (4, 5) parallèles et opposés, en matériau transparent au rayonnement infrarouge, qui définissent un passage pour la réception d'un échantillon d'huile;
une source (2, 3) de rayonnement infrarouge, disposée d'un côté du récipient, près d'un élément de paroi (4); et
un détecteur (10) de rayonnement infrarouge, disposé de l'autre côté du récipient, près de l'autre élément de paroi (5), caractérisé en ce que:
la source (2, 3) est constituée d'une lampe incandescente dont l'enveloppe en verre est ouverte à une extrémité, où l'enveloppe est fixée à l'élément de paroi (4) mentionné en premier, de sorte que celui-ci ferme l'enveloppe de verre.

2. Appareil selon la revendication 1, dans lequel les éléments de paroi (4, 5) du récipient (6) sont en fluorure de calcium.

3. Appareil selon la revendication 1 ou 2, comprenant un logement pour une source d'énergie électrique de faible tension destinée à l'alimentation de la source (2, 3) de rayonnement infrarouge.